# EUROPEAN PATENT SPECIFICATION

(11) **EP 3 875 448 B1**
(45) Date of publication and mention of the grant of the patent: **25.12.2024**
(21) Application number: 19878137.9
(22) Date of filing: 28.10.2019
(51) Int. Cl.: C08G 18/80, C07C 267/00, C08K 5/29, C08L 67/00, C08G 18/02, C08G 18/28, C08G 18/75, C08G 18/76, C08G 18/79, C08L 67/02, C08L 67/04

(54) **POLYCARBODIIMIDE COMPOUND, AND POLYESTER RESIN COMPOSITION AND POLYESTER RESIN MODIFIER IN WHICH SAME IS USED**
POLYCARBODIIMIDVERBINDUNG UND DIESE ENTHALTENDE POLYESTERHARZZUSAMMENSETZUNG UND POLYESTERHARZMODIFIKATOR
COMPOSÉ DE POLYCARBODIIMIDE, COMPOSITION DE RÉSINE DE POLYESTER ET MODIFICATEUR DE RÉSINE DE POLYESTER UTILISANT CELUI-CI

(30) Priority: 31.10.2018 JP 2018205562
(43) Date of publication of application: 08.09.2021
(73) Proprietor: Nisshinbo Chemical Inc., Tokyo 103-8650 (JP)
(72) Inventor: YANAGISAWA, Kenichi, Chiba-shi, Chiba 267-0056 (JP); SASAKI, Takahiro, Chiba-shi, Chiba 267-0056 (JP); KOTANI, Saori, Chiba-shi, Chiba 267-0056 (JP)
(74) Representative: Steffan & Kiehne Patentanwälte PartG mbB
(86) International application number: PCT/JP2019/042099
(87) International publication number: WO 2020/090702

(56) References cited:
- WO-A1-2017/006950
- JP-A- 2010 031 174
- JP-A- 2013 193 986
- JP-A- 2016 065 252
- JP-A- 2017 215 587
- JP-A- H0 827 092
- JP-A- H0 881 533
- JP-A- H09 309 871
- US-A1- 2016 017 122

## Description

### Technical Field

The present invention relates to a polycarbodiimide compound which can be preferably used for improving hydrolysis resistance of polyester resins and to a polyester resin composition and a polyester resin modifier in which the polycarbodiimide compound is used.

### Background Art

Polyester resins are generally excellent in transparency, mechanical strength, processability, solvent resistance, and the like. Therefore, polyester resins are widely used for fibers, films, sheets, and the like and are also utilized in recycling.

However, polyester resins are susceptible to hydrolysis due to degradation over time, and therefore, a carbodiimide compound is sometimes added thereto as a polyester resin modifier for the purpose of suppressing hydrolysis and improving hydrolysis resistance.

For example, PLT 1 and PTL 2 indicate that a certain urea-modified carbodiimide has good compatibility with polyester resins and is capable of improving hydrolysis resistance of polyester resins. Specifically, as a working example, a urea-modified carbodiimide to which urea bonds are introduced by di-n-butylamine (boiling point at 1 atm: 159°C) and which has the number of carbodiimide groups (degree of polymerization of carbodiimide groups) of 1 or 3 is disclosed. In addition, a urea-modified carbodiimide to which urea bonds are introduced by n-butylamine (boiling point at 1 atm: 78°C) and which has the number of carbodiimide groups (degree of polymerization of carbodiimide groups) of 10 is disclosed.

### Citation List

### Patent Literature

PLT1: JP 08-81533 A
PLT2: JP 08-27092 A

### Summary of Invention

### Technical Problem

As described above, the urea-modified carbodiimide disclosed in PLT 1 and PLT 2 is a urea-modified carbodiimide to which urea bonds are introduced by using an amine compound having a boiling point of as high as 150°C or higher, or a urea-modified carbodiimide having a degree of polymerization of carbodiimide groups of 10 when an amine compound having a boiling point lower than 150°C is used.

However, when an amine compound having a high boiling point is used as raw material, the unreacted amine compound is likely to remain in the obtained urea-modified carbodiimide without being distilled away. In addition, urea bonds are sometimes cleaved by heating during melt kneading with polyester resins, causing the amine compound to be liberated, and the amine compound may remain in a polyester resin composition including the urea-modified carbodiimide without vaporizing. Such a residual amine may adversely affect performance of polyester resins to which the urea-modified carbodiimide is added.

In addition, even in the case where n-butylamine, which has a boiling point lower than that of di-n-butylamine, is used as an amine compound, the urea-modified carbodiimide having a degree of polymerization of carbodiimide groups of 10 increases the melt viscosity of a polyester resin composition including the urea-modified carbodiimide and worsens processability.

Therefore, the polycarbodiimide compound to be added to polyester resins is required to have an amount of residual amine as low as possible and to have good processability in kneading, molding, without deteriorating hydrolysis resistance imparted to the polyester resins through addition of the polycarbodiimide compound.

The present invention has been made so as to solve the above problems and aims at providing a polycarbodiimide compound which has a small amount of residual amine and has good processability when the polycarbodiimide compound is added to polyester resin while keeping hydrolysis resistance of polyester resin imparted thereto through addition of the polycarbodiimide compound and at providing a polyester resin composition and polyester resin modifier using the polycarbodiimide compound.

### Solution to Problem

The present invention is based on the following finding: a polycarbodiimide compound in which terminal isocyanate groups are capped by an amine compound having a predetermined boiling point and which has a predetermined degree of polymerization of carbodiimide groups is excellent as a polyester resin modifier in terms of hydrolysis resistance and processability.

That is, the present invention provides the following [1] to [9], as defined in the appended claims.
[1] A polycarbodiimide compound represented by the following general formula (1):

   RₘNH₂₋ₘ-CO-NH-Z-(N=C=N-Z)ₙ-NH-CO-NH₂₋ₘRₘ (1)

   wherein Rₘ is a hydrocarbon residue of an amine compound which is represented by RₘNH₃₋ₘ and m is 1 or 2, and the amine compound being one or more selected from cyclohexylamine and diisopropylamine;
   Z is a residue obtained by removing two isocyanate groups from an aliphatic diisocyanate compound; and n is any integer of 2 to 7.
[2] The polycarbodiimide compound according to the above [1], wherein the aliphatic diisocyanate compound is one or more selected from dicyclohexylmethane-4,4'-diisocyanate, isophorone diisocyanate, and tetramethylxylylene diisocyanate.
[3] The polycarbodiimide compound according to any one of the above [1] to [2], wherein n is any integer of 3 to 6.
[4] A polyester resin modifier comprising the polycarbodiimide compound according to any one of the above [1] to [3].
[5] The polyester resin modifier according to the above [4], which is a compatibilizer for a polyester resin.
[6] The polyester resin modifier according to the above [5]. wherein the compatibilizer is a compatibilizer which compatibilizes a polyester resin (A) with a polyester resin (B) other than the polyester resin (A), and a difference between solubility parameters of the polyester resin (A) and the polyester resin (B) obtained by Fedors' method is 0.20 (cal/cm³)^{1/2} or more.
[7] The polyester resin modifier according to the above [5]. wherein the compatibilizer is a compatibilizer which compatibilizes a polyester resin with a polyamide resin.
[8] A polyester resin composition, comprising the polycarbodiimide compound according to any one of the above [1] to [3] and a polyester resin.
[9] The polyester resin composition according to the above [8], wherein a content of the polycarbodiimide compound is 0.2 to 5.0 parts by mass based on 100 parts by mass in total of the polyester resin.

### Advantageous Effects of Invention

The polycarbodiimide compound of the present invention has a small amount of residual amine derived from raw material and can be obtained with high quality. In addition, by virtue of adding the polycarbodiimide compound to polyester resins, good processability in kneading, molding is provided without deteriorating hydrolysis resistance imparted to the polyester resins.

Accordingly, a polyester resin composition using the polycarbodiimide compound has good hydrolysis resistance and is excellent in processability.

Further, according to the present invention, a polyester resin modifier capable of successfully imparting hydrolysis resistance to polyester resins is provided by using the polycarbodiimide compound.

### Description of Embodiments

Hereinafter, a polycarbodiimide compound, and a polyester resin composition and polyester resin modifier using the polycarbodiimide compound according to the present invention will be described in detail.

### [Polycarbodiimide compound]

The polycarbodiimide compound of the present invention is represented by the following general formula (1):

RₘNH₂₋ₘ-CO-NH-Z-(N=C=N-Z)ₙ-NH-CO-NH₂₋ₘRₘ (1)

wherein Rₘ is a hydrocarbon residue of an amine compound which is represented by RₘNH₃₋ₘ and m is 1 or 2, and the amine compound being one or more selected from cyclohexylamine and diisopropylamine;
Z is a residue obtained by removing two isocyanate groups from an aliphatic diisocyanate compound; and n is any integer of 2 to 7.

### (Amine compound)

The amine compound constitutes both terminals of the polycarbodiimide compound represented by formula (1) above, caps terminal isocyanate groups, and introduces urea bonds. The amine compound is represented by RₘNH₃₋ₘ, and m is 1 or 2. That is, the amine compound is a primary amine (RNH₂) or a secondary amine (R₂NH). R is a hydrocarbon group, and two Rs in the secondary amine may be the same or different from each other. In addition, Rs at both terminals represented in formula (1) above may be the same or different from each other.

The amine compound is a compound having a boiling point at 1 atm (hereinafter simply referred to as a "boiling point") of 150°C or lower, preferably having a boiling point of 80°C to 150°C, and more preferably having a boiling point of 80°C to 140°C.

When an amine compound having a high boiling point exceeding 150°C is used as raw material for synthesizing the polycarbodiimide compound, the unreacted amine compound is likely to remain in the polycarbodiimide compound without being distilled away. A polycarbodiimide compound with a large amount of residual amine may adversely affect performance of polyester resins to which the polycarbodiimide compound is added.

In addition, urea bonds in the polycarbodiimide compound are likely to be cleaved at about 150°C to 200°C, the urea bonds are also likely to be cleaved by, for example, heating during melt kneading with polyester resins, causing the amine compound to be liberated, and the liberated amine compound may remain without vaporizing.

In contrast, an amine compound with a boiling point of 150°C or lower is likely to vaporize, and when such an amine compound is used as raw material for synthesizing the polycarbodiimide compound, the amine compound is less likely to remain in the polycarbodiimide compound, and a high quality polycarbodiimide compound with a small amount of residual amine is obtained. In addition, a boiling point of 80°C or higher is preferable for obtaining sufficient reactivity in reaction for capping the terminal isocyanate groups at the time of synthesizing the polycarbodiimide compound.

Examples of the amine compound includes an aliphatic amine having a boiling point of 150°C or lower. Specific examples thereof include primary amines such as n-propylamine (boiling point: 49°C), n-butylamine (boiling point: 78°C), isobutylamine (boiling point: 63°C), sec-butylamine (boiling point: 63°C), tert-butylamine (boiling point: 46°C), and cyclohexylamine (boiling point: 135°C); and secondary amines such as diethylamine (boiling point: 55°C) and diisopropylamine (boiling point: 84°C). One of them may be used alone, or two or more thereof may be used in combination. Among these, from the viewpoint of, for example, ease of uniform mixing at the time of adding the polycarbodiimide compound to polyester resins, the amine compound is one or more selected from cyclohexylamine and diisopropylamine, and cyclohexylamine is preferable.

### (Aliphatic diisocyanate compound)

Z in formula (1) above is a residue obtained by removing two isocyanate groups from an aliphatic diisocyanate compound. A diisocyanate compound is a compound having two isocyanate groups.

The term "aliphatic diisocyanate compound" used herein means a diisocyanate compound which is not a compound in which carbon atoms directly bonded to isocyanate groups constitute an aromatic ring. That is, the hydrocarbon group bonded to isocyanate groups may be linear or cyclic and also includes a hydrocarbon group in which a carbon atom not directly bonded to an isocyanate group constitutes an aromatic group.

When Z described above is a compound in which carbon atoms directly bonded to isocyanate groups constitute an aromatic ring, that is, a compound derived from an aromatic isocyanate compound, the polycarbodiimide compound is less likely to impart sufficient hydrolysis resistance to polyester resins, polyester resins to which the polycarbodiimide compound is added has high viscosity, and processability in kneading, molding becomes poor.

Examples of the aliphatic diisocyanate compound include tetramethylene diisocyanate, hexamethylene diisocyanate, 1,4-cyclohexane diisocyanate, dicyclohexylmethane-4,4'-diisocyanate, methylcyclohexane diisocyanate, 1,3-bis(isocyanatomethyl)cyclohexane, 3-isocyanatomethyl-3,5,5-trimethylcyclohexyl isocyanate (another name: isophorone diisocyanate), xylylene diisocyanate, and 1,3-bis(2-isocyanato-2-propyl)benzene (another name: tetramethylxylylene diisocyanate). One of them may be used alone, or two or more thereof may be included. Among these, dicyclohexylmethane-4,4'-diisocyanate, isophorone diisocyanate, and tetramethylxylylene diisocyanate are preferable from the viewpoint of safety, the effect of improving hydrolysis resistance of polyester resin.

### (Degree of polymerization of carbodiimide groups)

The symbol n in formula (1) above represents the number of carbodiimide groups contained in the polycarbodiimide compound and is herein referred to as "degree of polymerization of carbodiimide groups".

The above symbol n is any integer of 2 to 7, preferably 3 to 6, and more preferably 4 to 6.

The polycarbodiimide compound added to polyester resins can impart hydrolysis resistance by virtue of its carbodiimide groups. When the above n is less than 2, sufficient hydrolysis resistance cannot be provided. In addition, when the above n is 7 or less, the polycarbodiimide compound is enabled to have adequate viscosity at a heating temperature in melt kneading with polyester resin, compatibility becomes good, and a uniform mixture with polyester resin is likely to be obtained. Accordingly, such a polycarbodiimide compound has good processability in kneading, molding.

### (Method for producing polycarbodiimide compound)

A method for producing the polycarbodiimide compound is not particularly limited and the polycarbodiimide compound can be produced by a known production method. For example, the polycarbodiimide compound can be produced by a production method comprising the step of subjecting the aliphatic diisocyanate compound to carbodiimidization reaction using a carbodiimidizing catalyst to obtain an isocyanate-terminated polycarbodiimide, and the step of performing reaction for capping a terminal isocyanate group of the isocyanate-terminated polycarbodiimide using the amine compound to obtain the polycarbodiimide compound. The method shown in Examples below is exemplified as a specific production method.

The carbodiimidizing catalyst has an action of promoting decarboxylation condensation reaction of the aliphatic diisocyanate compound. Examples thereof include an organic phosphorous compound such as a phospholene compound and a phosphoric ester compound; and an organometallic compound such as a metal alkoxide, a metal carbonyl complex, and a metal acetylacetonato complex. Phospholene oxides are preferable as the organic phosphorous compound from the viewpoint of catalytic activity. In addition, alkoxides of titanium, hafnium, zirconium are preferable as the organometallic compound.

Phospholene oxides are more preferable, and specific examples thereof include 3-methyl-1-phenyl-2-phospholene-1-oxide, 3-methyl-1-ethyl-1-phospholene-1-oxide, 1-phenyl-2-phospholene-1-oxide, 1-ethyl-2-phospholene-1-oxide, 1-methyl-2-phospholene-1-oxide, and 3-phospholene isomers thereof. Among these, 3-methyl-1-phenyl-2-phospholene-1-oxide is more preferable from the viewpoint of catalytic activity, availability.

An amount of the carbodiimidizing catalyst to be used for the carbodiimidization reaction may be a catalyst amount usually required to promote carbodiimidization reaction and is appropriately set according to the type of the diisocyanate compound, which is reaction raw material, the temperature and time for carbodiimidization reaction, the degree of polymerization of carbodiimide groups in a polycarbodiimide compound to be obtained. Usually, such an amount is 0.01 to 2.0 parts by mass, preferably 0.05 to 1.8 parts by mass, and more preferably 0.1 to 1.5 parts by mass with respect to 100 parts by mass of the aliphatic diisocyanate compound.

A reaction temperature for the carbodiimidization reaction is appropriately set according to adequate promotion of the reaction, the degree of polymerization of carbodiimide groups in a polycarbodiimide compound to be obtained. Usually, the reaction temperature is preferably 80°C to 220°C, more preferably 90°C to 200°C, and still more preferably 100°C to 195°C.

A reaction time for the carbodiimidization reaction is appropriately set according to reaction temperature, the degree of polymerization of carbodiimide groups in a polycarbodiimide compound to be obtained. Usually, the reaction time is preferably 1.0 to 36.0 hours, more preferably 2.0 to 30.0 hours, and still more preferably 3.0 to 25.0 hours.

A reaction temperature for reaction for capping a terminal isocyanate group of the isocyanate-terminated polycarbodiimide is appropriately set, according to the type of the amine compound used for capping within a range capable of promoting the reaction without causing side reaction. Usually, the reaction temperature is preferably 20°C to 200°C, more preferably 30°C to 190°C, and still more preferably 50°C to 180°C.

A reaction time for reaction for capping a terminal isocyanate group of the isocyanate-terminated polycarbodiimide is appropriately set according to reaction temperature, the type of the amine compound. Usually, the reaction time is preferably 0.1 to 3.0 hours, more preferably 0.2 to 2.0 hours, and still more preferably 0.3 to 1.5 hours.

### [Polyester resin composition]

The polyester resin composition of the present invention comprises the above-described polycarbodiimide compound and polyester resin.

From the viewpoint of performance required for its application, the polyester resin composition may comprises a known additive applied to polyester resins such as an antioxidant, a flame retardant, an ultraviolet absorber, and a colorant, for example, to the extent not impairing the effect of the present invention as needed.

The polyester resins are resins having, as a basic constitution, a polycondensate of a polycarboxylic acid and a polyhydric alcohol, a polycondensate of a hydroxy acid, and well-known polyester resins can be used.

Examples of the polyester resins include polyethylene terephthalate (PET), polybutylene terephthalate (PBT), polybutylene succinate (PBS), polybutylene succinate adipate (PBSA), polybutylene adipate terephthalate (PBAT), a polyhydroxyalkanoic acid (PHA) such as polylactic acid (PLA) and polyhydroxy butyric acid (PHB), polycaprolactone (PCL), polyethylene naphthalate, polyarylate, and an ethylene terephthalate-isophthalate copolymer. One of them may be used alone, or two or more thereof may be used in combination. Among these, PET, PBT, PBS, PBSA, PLA, and PHB are preferably used from the viewpoint of industrial availability and recyclability. From the viewpoint of biomass plastic, PLA, PHB are preferable, for example.

In the polyester resin composition, a content of the polycarbodiimide compound is preferably 0.2 to 5.0 parts by mass, more preferably 0.3 to 3.0 parts by mass, and still more preferably 0.5 to 2.0 parts by mass with respect to 100 parts by mass of the polyester resin.

When the content is 0.2 parts by mass or more, sufficient hydrolysis resistance can be imparted to the polyester resin. In addition, when the content is 5.0 parts by mass or less, deterioration in processability in kneading and molding caused by excessive addition of the polycarbodiimide compound can be prevented, and reduction in strength of a molded article formed from the polyester resin composition can be suppressed.

The polyester resin composition can be obtained by melting and kneading the polycarbodiimide compound and the polyester resin, for example. At this time, a mixture obtained by mixing the polycarbodiimide compound and the polyester resin in advance may be melted and kneaded, or the polycarbodiimide compound may be added to the polyester resin having been melted followed by kneading. Furthermore, a resin compound such as a masterbatch is once prepared, and the resin compound and polyester resin may be melted and kneaded by any of the above methods. It should be noted that the above-described additives may be added besides the polycarbodiimide compound to the extent not impairing the effect of the present invention.

Melting and kneading means is not particularly limited, and a known kneader can be used. Examples of the kneader include a single-screw extruder, a twin-screw extruder, and a rolling mixer.

Production of a polyester resin product using the polyester resin composition can be carried out by molding using a known method such as an injection molding method, a film molding method, a blow molding method, and a foam molding method. The polyester resin composition can be molded into various forms such as a film form, a sheet form, and a block form at a temperature equal to or higher than the melting point of the polyester resin used.

### [Polyester resin modifier]

A polyester resin modifier of the present invention includes the above-described polycarbodiimide compound.

As described above, the polycarbodiimide compound of the present invention is capable of successfully imparting hydrolysis resistance by adding the polycarbodiimide compound to polyester resins and therefore can be preferably used as a polyester resin modifier.

The polyester resin modifier includes a compatibilizer having a function of improving compatibility of polyester resins. At least one of resins to which the compatibilizer is added is polyester resins. The compatibilizer can successfully compatibilize different types of polyester resins with each other or successfully compatibilize polyester resins and polyamide resins.

A polyester resin composition with good compatibility can be obtained, also in a case where the polyester resin modifier is such a compatibilizer, by the same operation as melt kneading for obtaining the above-described polyester resin composition using different types of polyester resins or using polyester resins and polyamide resins.

Polyester resins to which the compatibilizer is added may be selected from the polyester resins listed in the description for the polyester resin composition.

In a case where a polyester resin composition is prepared by using different type of polyester resin in combination, compatibility is usually poor when a difference between solubility parameters (SP values) of polyester resin (A) and polyester resin (B), which are different from each other, obtained by Fedors' method is 0.20 (cal/cm³)^{1/2} or more. For example, PLA (11.10) and PBS (10.85), PLA (11.10) and PBSA (10.44 to 10.85), PET (12.39) and PLA (11.10), PLA (11.10) and PCL (10.16) are exemplified (the numerical values in the parentheses are SP values [(cal/cm³)^{1/2}]).

While the compatibilizer can be applied to any type of polyester resin, the compatibilizer is especially effective in improving compatibility between different type of polyester resin with the difference between SP values thereof is as large as 0.20 (cal/cm³)^{1/2} or more.

In addition, while examples of the polyamide resins are not particularly limited, examples thereof include nylon 6 and nylon 66 which are general-purpose resin. Even in a case where a polyester resin and a polyamide resin are used in combination to prepare a polyester resin composition, the compatibilizer can effectively improve compatibility therebetween.

Whether compatibility between different types of resins used in combination for a polyester resin composition to which the compatibilizer is added is good or poor can be determined using, as an index, a haze of a sheet-shaped molding (specimen) of the polyester resin composition. The haze can be measured by, in particular, the method described in Examples described later using a value measured by a method according to JIS K 7136:2000, that is, out of transmitted light passing through a specimen, a percentage of transmitted light that deviates from the incident light by 2.5° or more due to forward scattering. With a decrease in the value of the haze, the scattering of light becomes smaller, and the transmittance of the specimen becomes better. As such, compatibility between different types of resins used in combination for the polyester resin composition is considered to be good when the transmittance is good.

In a case where the polyester resin modifier is the compatibilizer, the polycarbodiimide compound in a polyester resin composition may also exert good compatibility at the same content as described above.

The polyester resin modifier may preliminarily include, in addition to the polycarbodiimide compound, the same additives described in the description for the polyester resin composition as appropriate according to application thereof to the extent not impairing the effect of the present invention. When such a polyester resin modifier is used in producing the polyester resin composition described above, effort for separately adding the additives can be saved, and work efficiency can be enhanced.

It should be noted that while characteristics of the modifier are not particularly limited, the modifier is preferably in solid form, especially, in powder or pellet form from the viewpoint of ease of handling.

### Examples

Hereinafter, the present invention will be described in detail with reference to examples.

### [Synthesis of polycarbodiimide compound]

Details of the raw material compounds used for synthesizing polycarbodiimide compounds in Examples and Comparative Examples described below are shown below.

### <Diisocyanate compound>

HMDI: dicyclohexylmethane-4,4'-diisocyanate; molecular weight: 262.35
TMXDI: tetramethylxylylene diisocyanate; molecular weight: 244.29
IPDI: isophorone diisocyanate; molecular weight: 222.29
MDI: 4,4'-diphenylmethanediisocyanate; molecular weight: 250.26

### <Amine compound>

CHA: cyclohexylamine; molecular weight: 99.18, boiling point: 135°C
DIPA: diisopropylamine; molecular weight: 101.19, boiling point: 84°C
BA: n-butylamine; molecular weight: 73.14, boiling point: 78°C
PA: n-propylamine; molecular weight: 59.11, boiling point: 49°C
DBA: di-n-butylamine; molecular weight: 129.24, boiling point: 159°C
DCHA: dicyclohexylamine; molecular weight: 181.32, boiling point: 256°C

Analyses and measurement in synthesizing polycarbodiimide compounds were conducted by the apparatuses or methods shown below.

### <Infrared (IR) spectrum measurement>

Apparatus used: Fourier transform infrared spectrophotometer "FTIR-8200PC" (manufactured by SHIMADZU CORPORATION)

### <Degree of polymerization of carbodiimide groups>

Apparatus used: automatic titrator "COM-900" (manufactured by HIRANUMA Co., Ltd.)

A toluene solution of DBA with a known concentration was mixed with an isocyanate-terminated polycarbodiimide obtained through polycarbodiimidization reaction, the terminal isocyanate group and DBA were reacted, the remaining DBA was subjected to neutralization titration with a hydrochloric acid standard solution, and the amount of remaining isocyanate groups (terminal NCO amount [mass%]) was calculated by a potentiometric titration method. The degree of polymerization n of carbodiimide groups was obtained from this terminal NCO amount.

### (Example 1)

To a reaction container equipped with a reflux condenser and a stirrer, 100 parts by mass of HMDI and 0.5 parts by mass of 3-methyl-1-phenyl-2-phospholene-1-oxide as a carbodiimidizing catalyst were added followed by stirring and mixing at 185°C for 6.5 hours under nitrogen stream to conduct carbodiimidization reaction to obtain an isocyanate-terminated polycarbodiimide.

With respect to the obtained isocyanate-terminated polycarbodiimide, an absorption peak around the wavelength of 2150 cm⁻¹ derived from the carbodiimide groups was observed by IR spectrum measurement. In addition, the terminal NCO amount was 12.02% by mass, and the degree of polymerization of carbodiimide groups was 2.

Thereafter, under nitrogen stream, 25.2 parts by mass (an amount equivalent to the amount of terminal isocyanate groups in the isocyanate-terminated polycarbodiimide in terms of mole) of CHA was added to the isocyanate-terminated polycarbodiimide at 150°C followed by stirring and mixing for 0.5 hours to conduct capping reaction for terminal isocyanate groups.

After the absorption peak at the wavelength of 2200 to 2300 cm⁻¹ derived from the isocyanate groups was confirmed to have disappeared by IR spectrum measurement, the reaction product was taken out of the reaction container and cooled to room temperature to obtain a light yellow transparent solid polycarbodiimide compound.

### (Examples 2 to 10 and Comparative Examples 1 to 8)

Each polycarbodiimide compound having the predetermined degree of polymerization n shown in Table 1 below was synthesized in the same manner as in Example 1 except that the diisocyanate compound, amine compound, and reaction conditions (temperature and time) of carbodiimidization reaction were respectively changed as shown in Table 1 below in Example 1.

### (Comparative Example 9)

To a reaction container equipped with a reflux condenser and a stirrer, 100 parts by mass of MDI and 13.2 parts by mass of CHA were added followed by stirring and mixing at room temperature (25°C) for 0.5 hours under nitrogen stream to conduct capping reaction for terminal isocyanate groups of MDI. Thereafter, 0.5 parts by mass of 3-methyl-1-phenyl-2-phospholene-1-oxide was added as a carbodiimidizing catalyst followed by stirring and mixing at 110°C for 2.0 hours to conduct carbodiimidization reaction, and a light yellow transparent solid polycarbodiimide compound was obtained.

### [Preparation of polyester resin composition]

Each polyester resin composition was prepared using the polycarbodiimide compound synthesized in each of Examples and Comparative Examples described above and polyester resins (and a polyamide resin) shown below.

### <Polyester resin>

PBSA: polybutylene succinate adipate; "BioPBS (R) FD-92PM," manufactured by PTT MCC Biochem Co., Ltd.
PLA: polylactic acid; "Ingeo (R) biopolymer 4032D," manufactured by Nature Works LLC
PBS: polybutylene succinate
PET: polyethylene terephthalate; "TRN-8550FF," manufactured by TEIJIN LIMITED

### <Polyamide resin>

Ny6: nylon 6; "UNITIKA nylon 6 A1030BRL," manufactured by UNITIKA LTD.

### (Preparation of polyester resin composition (1))

After 100 parts by mass of PBSA was melted at 170°C using a laboratory mixer ("segment mixer KF70V," manufactured by Toyo Seiki Seisaku-sho, Ltd., LABO PLASTOMILL (R); the same applies hereinafter), 1.0 parts by mass of the polycarbodiimide compound was added thereto followed by kneading for three minutes to prepare polyester resin composition (1).

### (Preparation of polyester resin composition (2))

After 90 parts by mass of PLA and 10 parts by mass of PBSA were melted at 210°C using a laboratory mixer, 0.5 parts by mass of the polycarbodiimide compound was added thereto followed by kneading for three minutes to prepare polyester resin composition (2) (PET/PBSA).

### (Preparation of polyester resin composition (3))

After 80 parts by mass of PLA and 20 parts by mass of PBS were melted at 210°C using a laboratory mixer, 0.5 parts by mass of the polycarbodiimide compound was added thereto followed by kneading for three minutes to prepare polyester resin composition (3) (PET/PBS).

### (Preparation of polyester resin composition (4))

After 80 parts by mass of PET and 20 parts by mass of Ny6 were melted at 260°C using a laboratory mixer, 0.5 parts by mass of the polycarbodiimide compound was added thereto followed by kneading for three minutes to prepare polyester resin composition (4) (PET/Ny6).

### [Evaluation of polycarbodiimide compound and polyester resin composition]

Each of the polycarbodiimide compounds and polyester resin compositions obtained above was evaluated in terms of the following items. Evaluation results thereof are summarized and shown in Table 1 below.

### (Amount of residual amine)

The polycarbodiimide compound was dissolved in tetrahydrofuran and subsequently mixed with acetonitrile to precipitate the polycarbodiimide compound followed by filtration. The quantity of the unreacted amine compound remaining in the filtrate was determined by high performance liquid chromatography (HPLC). Measurement conditions of HPLC are as follows.

### <Measurement conditions>

Column: ACQUITY UPLC BEH C18 (manufactured by Waters Corporation, inner diameter 2.1 mm × length 100 mm, particle diameter: 1.7 µm)
Column temperature: 40°C
Mobile phase: formic acid/methanol = 0.1/99.9 (volume ratio), flow rate: 0.4 mL/min
Detector: MS/MS (tandem mass spectrometry)

In Table 1 below, evaluation results in which the case where the amount of residual amine is less than 20 ppm is designated as "A", and the case where the amount of residual amine is 20 ppm or more is designated as "B" are shown.

### (Hydrolysis resistance)

The polyester resin composition (1) was molded into a sheet form with a thickness of about 300 µm by hot pressing at 170°C, and a strip-shaped specimen with a width of 10 mm and a length of 10 cm was subsequently prepared.

A tensile test was conducted immediately after the preparation (early stage) and after damp heat treatment. The damp heat treatment was carried out by exposing the specimen to a temperature of 70°C and relative humidity of 90% for 200 hours using a damp heat tester.

The tensile test was carried out by measuring tensile elongation at breakage of the specimen under conditions of a gauge length of 30 mm and tensile speed of 100 mm/min using a tensile tester ("3365" manufactured by Instron Corporation). The relative ratio of the tensile elongation after damp heat treatment was calculated based on the tensile elongation at the early stage as 100.

With an increase in the relative ratio of tensile elongation, the degree of decrease in tensile elongation before and after damp heat treatment becomes smaller, and hydrolysis resistance can be said to be excellent.

In Table 1 below, evaluation results in which the case where the relative ratio of tensile elongation is 80 or more is designated as "A", the case where the relative ratio of tensile elongation is 60 or more and less than 80 is designated as "B", and the case where the relative ratio of tensile elongation is less than 60 is designated as "C" are shown. Incidentally, as a comparative reference, the same tensile test as described above was also conducted on the case where no polycarbodiimide compound was added, and the evaluation result thereof was "C".

### (Processability)

The melt viscosity of polyester resin composition (1) was measured at 170°C by a capillary rheometer ("flow tester CFT-500D," manufactured by SHIMADZU CORPORATION) using an orifice with a diameter of 1.0 mm × 10.0 mm.

It can be said that with a decrease in the melt viscosity, the processability in uniformly mixing polyester resin composition (1) becomes better, and molding is easier, and processability is excellent.

In Table 1 below, evaluation results in which the case where the melt viscosity is less than 1,200 Pa·s is designated as "A", the case where the melt viscosity is 1,200 Pa·s or more and less than 1,600 Pa·s is designated as "B", and the case where the melt viscosity is 1,600 Pa·s or more is designated as "C" are shown.

### (Compatibility)

Each of obtained polyester resin compositions (2) to (4) was molded into a sheet form with a thickness of 150 to 200 µm by hot pressing to prepare a specimen (50 mm × 50 mm). The hot pressing temperature was set to 210°C for polyester resin compositions (2) and (3) and to 260°C for polyester resin composition (4).

In addition, each blank specimen was prepared in the same manner as the above-described specimen using each polyester resin composition obtained by conducting the same operation except that the polycarbodiimide compound was not added in preparing polyester resin compositions (2) to (4).

The haze of each of the specimens and blank specimens was measured by a method according to JIS K 7136:2000 using a haze meter ("NDH5000," manufactured by NIPPON DENSHOKU INDUSTRIES CO., LTD.).

As a value of the haze decreases, light scattering becomes smaller, and transmittance of the specimen becomes better. When compatibility between two types of resins among polyester resin compositions (2) to (4) is good, transmittivity of the specimen is good, and the haze value decreases. Therefore, the haze value was used as an index of compatibility.

It can be said that with an increase in the difference (ΔH) obtained by subtracting the haze value of the specimen from the haze value (reference value) of the blank specimen, the effect of the added polycarbodiimide compound as a compatibilizer is superior.

In Table 1 below, evaluation results in which the case where the ΔH is 10% or more is designated as "A", the case where the ΔH is 5% or more and less than 10% is designated as "B", and the case where the ΔH is less than 5% is designated as "C" are shown. Examples 9 and 10 are reference examples.

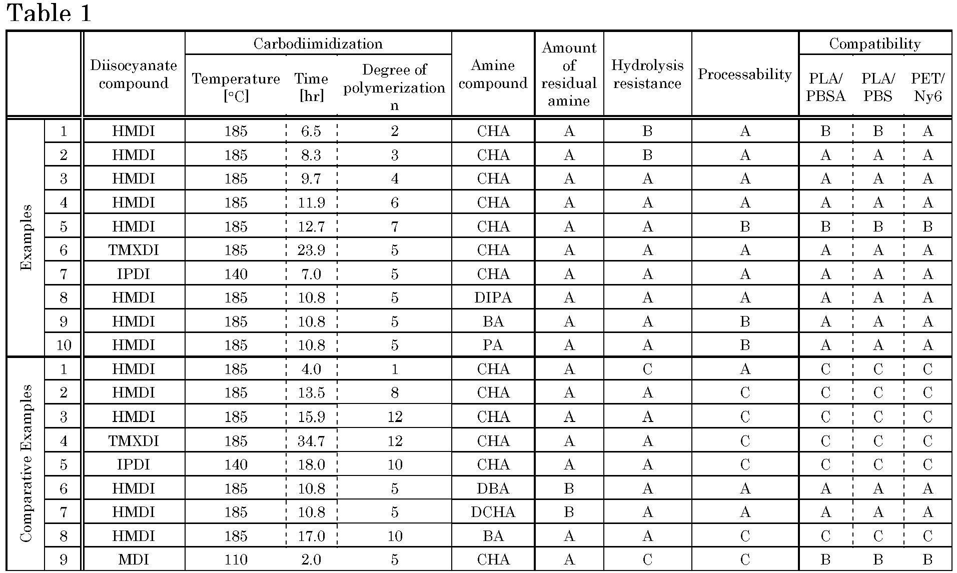

As seen from the evaluation results in Table 1, it can be said that the obtained polycarbodiimide compound according to the present invention has a small amount of residual amine derived from raw material, and the quality thereof is high.

In addition, the result of hydrolysis resistance for polyester resin composition (1) obtained by using the polycarbodiimide compound is also good, processability thereof is also excellent, and it has been confirmed that the polycarbodiimide compound provides a good effect as a polyester resin modifier.

In addition, it has been also confirmed that the polycarbodiimide compound can improve compatibility between different types of resins in a polyester resin composition and provides a good effect as a compatibilizer for polyester resin.

## Claims

1. A polycarbodiimide compound represented by the following general formula (1):
RₘNH₂₋ₘ-CO-NH-Z-(N=C=N-Z)ₙ-NH-CO-NH₂₋ₘRₘ (1)
wherein Rₘ is a hydrocarbon residue of an amine compound which is represented by RₘNH₃₋ₘ and m is 1 or 2, and the amine compound being one or more selected from cyclohexylamine and diisopropylamine;
Z is a residue obtained by removing two isocyanate groups from an aliphatic diisocyanate compound; and
n is any integer of 2 to 7, and n being determined according to the method specified in the specification.

2. The polycarbodiimide compound according to claim 1, wherein the aliphatic diisocyanate compound is one or more selected from dicyclohexylmethane-4,4'-diisocyanate, isophorone diisocyanate, and tetramethylxylylene diisocyanate.

3. The polycarbodiimide compound according to claim 1 or 2, wherein n is any integer of 3 to 6.

4. A polyester resin modifier, comprising the polycarbodiimide compound according to any one of claims 1 to 3.

5. The polyester resin modifier according to claim 4, which is a compatibilizer for a polyester resin.

6. The polyester resin modifier according to claim 5, wherein the compatibilizer is a compatibilizer which compatibilizes a polyester resin (A) with a polyester resin (B), and a difference between solubility parameters of the polyester resin (A) and the polyester resin (B) obtained by Fedors' method is 0.41 MPa^{1/2} (0.20 (cal/cm³)^{1/2}) or more.

7. The polyester resin modifier according to claim 5, wherein the compatibilizer is a compatibilizer which compatibilizes a polyester resin with a polyamide resin.

8. A polyester resin composition, comprising the polycarbodiimide compound according to any one of claims 1 to 3 and a polyester resin.

9. The polyester resin composition according to claim 8, wherein a content of the polycarbodiimide compound is 0.2 to 5.0 parts by mass based on 100 parts by mass in total of the polyester resin.

## Patentansprüche

1. Polycarbodiimid-Verbindung, dargestellt durch die folgende allgemeine Formel (1):
RₘNH₂₋ₘ-CO-NH-Z-(N=C=N-Z)ₙ-NH-CO-NH₂₋ₘRₘ (1)
wobei Rₘ ein Kohlenwasserstoffrest einer Amin-Verbindung ist, die durch RₘNH₃₋ₘ dargestellt wird, und m 1 oder 2 ist, und die Amin-Verbindung eine oder mehrere ausgewählt aus Cyclohexylamin und Diisopropylamin ist;
Z ein Rest ist, der durch Entfernung zweier Isocyanat-Gruppen von einer aliphatischen Diisocyanat-Verbindung erhalten wird; und
n eine beliebige ganze Zahl von 2 bis 7 ist, und n gemäß dem in der Beschreibung angegebenen Verfahren bestimmt wird.

2. Polycarbodiimid-Verbindung nach Anspruch 1, wobei die aliphatische Diisocyanat-Verbindung eine oder mehrere ausgewählt aus Dicyclohexylmethan-4,4'-diisocyanat, Isophoron-diisocyanat und Tetramethylxylylen-diisocyanat ist.

3. Polycarbodiimid-Verbindung nach Anspruch 1 oder 2, wobei n eine beliebige ganze Zahl von 3 bis 6 ist.

4. Polyesterharz-Modifikator, umfassend die Polycarbodiimid-Verbindung nach einem der Ansprüche 1 bis 3.

5. Polyesterharz-Modifikator nach Anspruch 4, der ein Kompatibilisator für ein Polyesterharz ist.

6. Polyesterharz-Modifikator nach Anspruch 5, wobei der Kompatibilisator ein Kompatibilisator ist, der ein Polyesterharz (A) mit einem Polyesterharz (B) kompatibel macht, und eine Differenz zwischen Löslichkeitsparametern des Polyesterharzes (A) und des Polyesterharzes (B), erhalten durch das Verfahren nach Fedors, 0,41 MPa^{1/2} (0,20 (cal/cm³) ^{1/2}) oder mehr beträgt.

7. Polyesterharz-Modifikator nach Anspruch 5, wobei der Kompatibilisator ein Kompatibilisator ist, der ein Polyesterharz mit einem Polyamidharz kompatibel macht.

8. Polyesterharz-Zusammensetzung, umfassend die Polycarbodiimid-Verbindung nach einem der Ansprüche 1 bis 3 und ein Polyesterharz.

9. Polyesterharz-Zusammensetzung nach Anspruch 8, wobei ein Gehalt der Polycarbodiimid-Verbindung 0,2 bis 5,0 Massenteile bezogen auf insgesamt 100 Massenteile des Polyesterharzes beträgt.

## Revendications

1. Composé polycarbodiimide, représenté par la formule générale (1) suivante :
RₘNH₂₋ₘ-CO-NH-Z-(N=C=N-Z)ₙ-NH-CO-NH₂₋ₘRₘ (1)
dans lequel Rₘ est un résidu hydrocarbure d'un composé aminé représenté par RₘNH₃₋ₘ, et m est 1 ou 2, et le composé aminé est un ou plusieurs sélectionné parmi la cyclohexylamine et la diisopropylamine;
Z est un résidu obtenu par l'enlèvement de deux groupes isocyanate d'un composé diisocyanate aliphatique ; et
n est un nombre entier quelconque de 2 à 7, et n est déterminée selon le procédé spécifié dans la spécification.

2. Composé polycarbodiimide selon la revendication 1, dans lequel le composé diisocyanate aliphatique est un ou plusieurs sélectionné parmi le diisocyanate-4,4' de dicyclohexylméthane, le diisocyanate d'isophorone et le diisocyanate de tétraméthylxylylène.

3. Composé polycarbodiimide selon la revendication 1 ou 2, dans lequel n est un nombre entier quelconque de 3 à 6.

4. Modificateur de résine de polyester, comprenant le composé polycarbodiimide selon l'une quelconque des revendications 1 à 3.

5. Modificateur de résine de polyester selon la revendication 4, étant un compatibilisant pour une résine de polyester.

6. Modificateur de résine de polyester selon la revendication 5, dans lequel le compatibilisant est un compatibilisant qui rend une résine de polyester (A) compatible avec une résine de polyester (B), et une différence entre les paramètres de solubilité de la résine de polyester (A) et la résine de polyester (B) comme obtenue par la méthode de Fedors est 0,41 MPa^{1/2} (0,20 (cal/cm³) ^{1/2}) ou supérieure.

7. Modificateur de résine de polyester selon la revendication 5, dans lequel le compatibilisant est un compatibilisant qui rend une résine de polyester compatible avec une résine de polyamide.

8. Composition de résine de polyester comprenant le composé polycarbodiimide selon l'une quelconque des revendications 1 à 3 et une résine de polyester.

9. Composition de résine de polyester selon la revendication 8, dans laquelle une teneur du composé polycarbodiimide est de 0,2 à 5,0 parties en masse par rapport à un total de 100 parties en masse de la résine de polyester.
